(19) Europäisches Patentamt
European Patent Office
Office européen des brevets

(11) Veröffentlichungsnummer: **0 008 057**
**B1**

(12) # EUROPÄISCHE PATENTSCHRIFT

(45) Veröffentlichungstag der Patentschrift: **12.05.82**

(21) Anmeldenummer: **79102706.3**

(22) Anmeldetag: **30.07.79**

(51) Int. Cl.³: **C 07 D 231/12**, C 07 D 307/54, C 07 D 333/20, C 07 D 249/02, C 07 D 257/04, C 07 D 271/10, C 07 D 285/12 // A01N43/00

(54) **Verfahren zur Herstellung von N-substituierten alpha-Halogen-acetaniliden.**

(30) Priorität: **10.08.78 DE 2835157**

(43) Veröffentlichungstag der Anmeldung: **20.02.80 Patentblatt 80/4**

(45) Bekanntmachung des Hinweises auf die Patenterteilung: **12.05.82 Patentblatt 82/19**

(84) Benannte Vertragsstaaten: **AT BE CH DE FR GB IT NL SE**

(56) Entgegenhaltungen:
**DE-A1-2 [illegible]**
**DE-A1-2 704 281**
**FR-A-2 217 328**
**US-A-3 901 917**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Schmidt, Thomas, Dr., Am Bandenfeld 72, D-5657 Haan (DE)**
Erfinder: **Stetter, Jörg, Dr., Pahlkestrasse 3, D-5600 Wuppertal 1 (DE)**
Erfinder: **Thomas, Rudolf, Dr., Wilkhausstrasse 129, D-5600 Wuppertal 2 (DE)**

**EP 0 008 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von N-substituierten α-Halogenacetaniliden

Die vorliegende Erfindung betrifft ein neues, vorteilhaftes Verfahren zur Herstellung von weitgehend bekannten N-substituierten α-Halogenacetaniliden, welche herbizide und fungizide Eigenschaften aufweisen.

Es ist bereits bekanntgeworden, daß man N-substituierte α-Halogenacetanilide erhält, wenn man in einer ersten Stufe Aniline mit entsprechend substituierten Halogenmethyl-Derivaten in Gegenwart eines inerten organischen Lösungsmittels und in Gegenwart eines Säurebindemittels vorzugsweise bei der Siedetemperatur des verwendeten Lösungsmittels umsetzt und die so erhaltenen N-substituierten Aniline in einer zweiten Stufe in Gegenwart eines inerten organischen Lösungsmittels und gegebenenfalls in Gegenwart eines Säurebindemittels bei Temperaturen zwischen 20 und 100°C mit einem Halogenacetylierungsmittel, wie beispielsweise Halogenacetylchlorid, gemäß dem folgenden Reaktionsschema umsetzt (vgl. unter anderem US-Patentschriften 3 442 945, 3 780 090, 3 900 497, 3 946 045, 3 948 950, 4 001 325, 4 032 657 und 3 901 917, französische Patentschrift 2 217 328 sowie deutsche Offenlegungsschriften 2 305 495, 2 311 897, 2 328 340, 2 350 944, 2 402 983, 2 648 008 und 2 704 281):

Reaktionsschema 1:

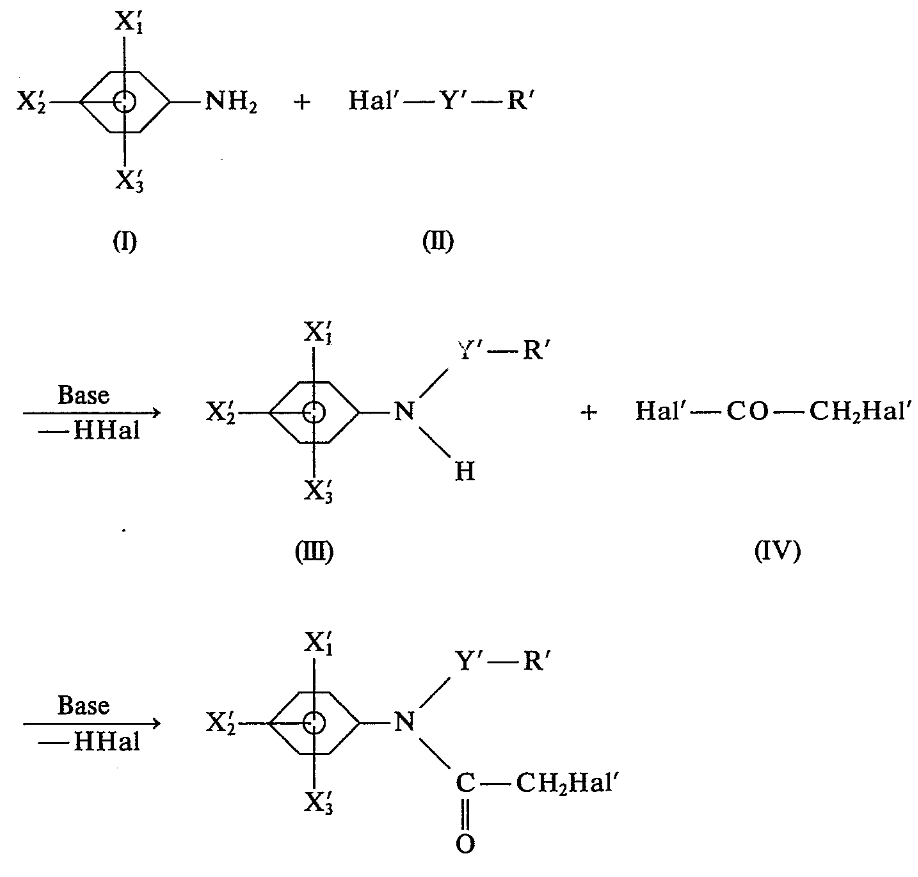


(I) (II) (III) (IV) (V)

R' = Alkoxy, Alkyl, Alkenyl, Alkinyl, Dioxoanyl, Dithiepanyl, über ein Ring-Stickstoffatom gebundener, N-haltiger heterocyclischer Rest, Carbonsäureester-Rest u. a.,

$X_1$, $X_2'$, $X_3'$ = Wasserstoff, Alkyl, Alkoxy, Halogenalkyl, Alkoxyalkyl u. a., wobei $X_1'$, $X_2'$ und $X_3'$ gleichartig oder verschieden sind,

Y' = Alkylen, Alkyliden,

Hal' = Halogen.

Dieses Verfahren weist jedoch eine Reihe von Nachteilen auf. So ist die Gefahr für Nebenreaktionen in beiden Verfahrensschritten sehr groß, was zu erheblichen Ausbeuteverlusten führen kann. Zum Beispiel kann das Halogenacetylierungsmittel nicht nur an dem Stickstoff des Anilinteils, sondern auch an anderen reaktiven Positionen des Moleküls angreifen. Dieses ist vor allem dann der Fall, wenn der in dem obigen Reaktionsschema als R' bezeichnete Rest für einen Azolyl-Rest steht. Die

Halogenacetylierung kann dann auch an einem N-Atom des Restes R' stattfinden. Ferner besteht bei der Alkylierung der Aniline in der ersten Stufe die Möglichkeit, daß beide Wasserstoffatome des Anilin-Stickstoffes reagieren, was zu Substanzgemischen führt.

Weiterhin ist bereits bekanntgeworden, daß man insbesondere N-Azolylmethyl-α-halogenacetanilide erhalten kann, wenn man Aniline mit Paraformaldehyd in Gegenwart katalytischer Mengen Kaliumhydroxid umsetzt, die entstehenden Phenylazomethine mit einem Halogenacethalogenid versetzt (vgl. US-Patentschriften 3 630 716 und 3 637 847) und die entstehenden N-Halogenmethyl-α-halogenacetanilide mit entsprechenden Heterocylen, gegebenenfalls in Gegenwart eines Säurebindemittels und in Gegenwart eines inerten organischen Lösungsmittels bei Temperaturen zwischen 0 und 200° C gemäß dem folgenden Reaktionsschema umsetzt (vgl. auch DE-OS 2 648 008):

Reaktionsschema 2:

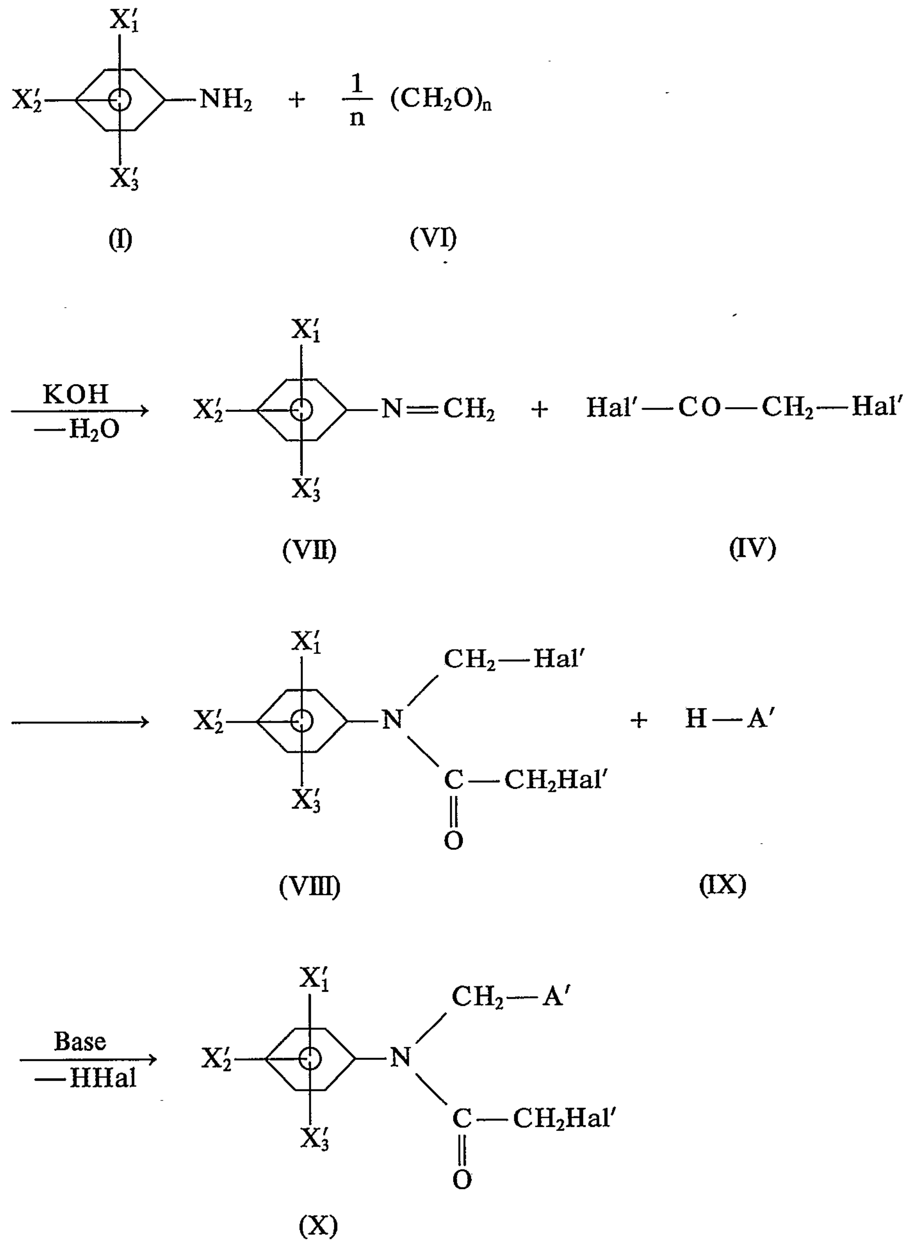


(I) (VI)

(VII) (IV)

(VIII) (IX)

(X)

A' = über ein Ringstickstoffatom gebundener N-haltiger heterocyclischer Rest

$X_1'$, $X_2'$, $X_3'$ und Hal' = siehe oben

Auch dieses Verfahren hat den Nachteil, daß es nicht immer in befriedigenden Ausbeuten abläuft. Die auftretenden Zwischenprodukte sind nicht in allen Fällen sehr stabil und lassen sich wegen ihrer hohen Reaktivität nur schlecht in reiner Form isolieren, was häufig zu stark verunreinigten

Endprodukten führt. Da außerdem bei der technischen Herstellung der Stoffe der Formel (X) in den als Zwischenprodukte anfallenden Azomethinen immer noch Formaldehyd (als Paraformaldehyd) enthalten ist, bilden sich bei der Umsetzung mit Halogenacetylhalogeniden tränenreizende und stark cancerogene Bishalogenmethylether.

Es wurde gefunden, daß die weitgehend bekannten N-substituierten $\alpha$-Halogenacetanilide der Formel

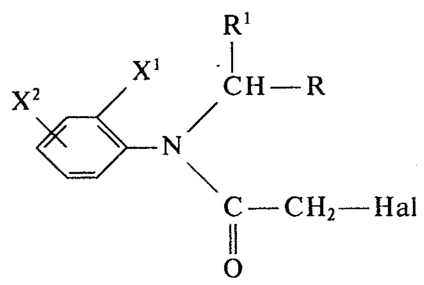

(XI)

in welcher

R für Pyrazol-1-yl, 1,2,4-Triazol-1-yl, 1,2,3-Triazol-1-yl, 1,3,4-Triazol-1-yl, 1,2,3,4-Tetrazol-1-yl oder Pyrrol-1-yl steht, wobei jeder dieser Reste substituiert sein kann durch Halogen und/oder Alkyl mit 1 bis 4 Kohlenstoffatomen, und

R ferner für die Gruppierung der Formel

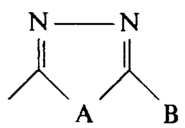

steht, in welcher

A für Sauerstoff, Schwefel oder $>NR^2$ steht,
worin
$R^2$ für Wasserstoff, Alkyl mit 1 bis 4 Kohlenstoffatomen oder Phenyl steht, und

B für Wasserstoff, Alkyl mit 1 bis 4 Kohlenstoffatomen, Halogenalkyl mit bis zu 3 Kohlenstoffatomen und bis zu 5 gleichen oder verschiedenen Halogenatomen, Halogen, Phenyl, Benzyl oder für die Gruppierungen $-OR^3$, $-SR^3$ oder $-NR^2R^3$ steht,
worin
$R^2$ die oben angegebenen Bedeutungen hat und
$R^3$ für Wasserstoff oder Alkyl mit 1 bis 4 Kohlenstoffatomen steht,
und

R außerdem für Furyl, Thiophenyl, Alkenyl mit 2 bis 4 Kohlenstoffatomen, Alkinyl mit 2 bis 4 Kohlenstoffatomen, Alkoxy mit 1 bis 4 Kohlenstoffatomen, Alkylcarbonyl mit 1 bis 4 Kohlenstoffatomen im Alkylteil oder für Phenyl, Phenoxy oder Phenylcarbonyl steht, wobei jeder der drei letztgenannten Reste substituiert sein kann durch Halogen und/oder Alkyl mit 1 bis 4 Kohlenstoffatomen,

$R^1$ für Wasserstoff, Alkyl mit 1 bis 4 Kohlenstoffatomen oder Phenyl steht,

$X^1$ für Alkyl mit 1 bis 4 Kohlenstoffatomen steht,

$X^2$ für Wasserstoff oder Alkyl mit 1 bis 4 Kohlenstoffatomen steht, und

Hal für Chlor oder Brom steht,

erhält, wenn man Halogenacetanilide der Formel

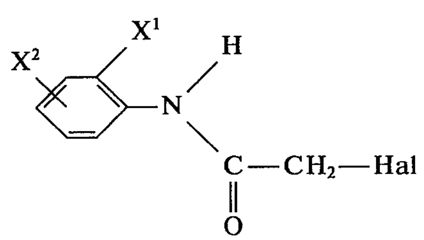

(XII)

in welcher

$X^1$, $X^2$
und Hal die oben angegebene Bedeutung haben,

mit Verbindungen der Formel

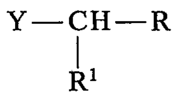

(XIII)

in welcher

R und R[1] die oben angegebene Bedeutung haben und

Y für Chlor, Brom, Jod, Alkylsulfonyl mit 1 bis 4 Kohlenstoffatomen oder für gegebenenfalls durch Halogen oder Alkyl mit 1 bis 4 Kohlenstoffatomen substituiertes Phenylsulfonyl steht,

in Gegenwart eines Alkalihydroxids oder eines Alkalicarbonats als Säurebinder und gegebenenfalls in Gegenwart eines organischen Lösungsmittels bei Temperaturen zwischen −20°C und +100°C umsetzt, wobei die Verbindungen der Formel (XIII) gegebenenfalls in Form von Säureadditions-Salzen umgesetzt werden können.

Es ist als ausgesprochen überraschend zu bezeichnen, daß die erfindungsgemäße Umsetzung in der angegebenen Art und Weise abläuft. Dabei kommt es unter den vorgegebenen Verfahrensbedingungen zur Bildung eines Anions am Stickstoffatom der Halogenacetanilide der Formel (XII), welches dann mit den Verbindungen der Formel (XIII) gut zu den erfindungsgemäßen Stoffen der Formel (XI) reagiert. Es bestehen jedoch eine Reihe von Reaktionsmöglichkeiten, die viel wahrscheinlicher erscheinen. Zu erwarten wäre z. B., daß das obengenannte Anion intramolekular mit der Halogenmethylgruppe zu einem Dreiring reagiert oder daß es über die mögliche tautomere Form

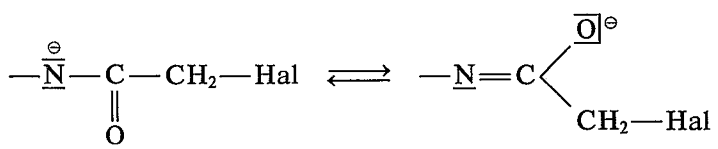

zur Epoxidbildung kommt. Zu erwarten wäre auch eine intermolekulare Nebenreaktion, d. h., daß anstatt einer N-Alkylierung am Halogenacetanilid eine O-Alkylierung an der obengenannten tautomeren Form stattfindet.

Außerdem geht aus der Publikation in Journal für praktische Chemie 40, 425—444 (1889), hervor, daß Anilin-Derivate, die am Stickstoffatom eine Halogenacetyl-Gruppe enthalten, in Gegenwart von Basen leicht zu 2,5-Dioxo-1,4-diphenylpiperazinen dimerisieren. Daher war zu erwarten, daß ebenso im Falle der erfindungsgemäßen Umsetzung eine solche Dimerisierung der Stoffe der Formel (XII) nach folgendem Schema ablaufen würde:

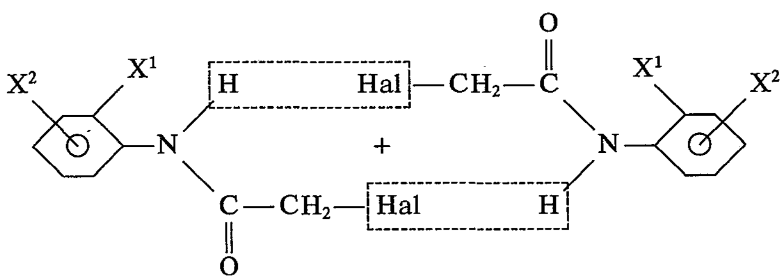

(XII) (XIV)

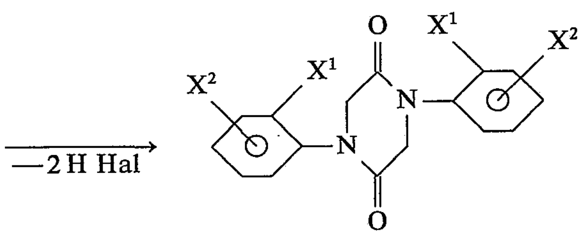


(XIV)

Im Gegensatz zu dieser Annahme führt das erfindungsgemäße Verfahren jedoch in glatter Reaktion zu den gewünschten Produkten. Ein derartiger Verlauf der Umsetzung war auch unter Berücksichtigung der technischen Lehre, die der DE-A-2 648 008, DE-A-2 704 281, FR-A-2 217 328 und

der US-A-3 901 917 zu entnehmen ist, nicht vorauszusehen, denn bei keinem der dort beschriebenen Verfahren zur Synthese von N-substituierten Halogenacetaniliden wird eine Ausgangsverbindung verwendet, die zur Dimerisation in der Lage wäre. Das vorhandene technische Vorurteil gegen die Durchführbarkeit des erfindungsgemäßen Verfahrens wird also durch die genannte Literatur nicht beseitigt.

Im übrigen ist die Substanzklasse der Acetanilide in den letzten Jahren wegen der guten herbiziden Wirksamkeit dieser Stoffe sehr intensiv bearbeitet worden. Dennoch wird bei keinem der zahlreichen publizierten Verfahren zur Synthese von N-substituierten Acetaniliden eine Ausgangsverbindung verwendet, die am Stickstoffatom neben Wasserstoff noch eine Halogenacetyl-Gruppe enthält und deshalb zu einem Piperazin-Derivat dimerisieren könnte.

Schließlich wäre bei der Durchführung des erfindungsgemäßen Verfahrens bei Verwendung von wäßriger Natronlauge als Säurebinder auch ein nucleophiler Austausch des Halogens sowohl bei den Halogenacetaniliden der Formel (XII) als auch bei den Verbindungen der Formel (XIII) gegen die Hydroxygruppe denkbar.

Aufgrund dieser möglichen und auch wahrscheinlichen Reaktionsabläufe ist der komplikationslose Verlauf der erfindungsgemäßen Umsetzung um so überraschender.

Das erfindungsgemäße Verfahren weist eine Reihe von Vorteilen auf. So sind sowohl die als Ausgangsstoffe zu verwendenden Halogenacetanilide der Formel (XII) als auch die Verbindungen der Formel (XIII) leicht zugänglich und als stabile Substanzen nahezu quantitativ und rein isolierbar. Die Bedingungen bei der Durchführung des erfindungsgemäßen Verfahrens sind sehr milde, insbesondere werden nur sehr niedrige Temperaturen benötigt. Die erfindungsgemäßen Verbindungen werden in guter Ausbeute und hoher Reinheit erhalten. Besonders hervorzuheben ist außerdem die breite Anwendbarkeit dieses Verfahrens, da grundsätzlich alle solche Verbindungen der Formel (XIII) einsetzbar sind, bei denen der Rest A den Austausch der Abgangsgruppe Y aktiviert.

Gegenüber den vorbekannten Methoden zur Synthese von Halogenacetaniliden zeichnet sich das erfindungsgemäße Verfahren jedoch nicht dadurch aus, daß sich die Endprodukte in höheren Ausbeuten als bisher darstellen lassen. Vielmehr sind diese Substanzen problemloser zugänglich als bisher. So entfällt die bei der Herstellung nach dem Reaktionsschema (1) auftretende Nebenreaktion, bei der das Halogenacetylierungsmittel nicht an dem Stickstoff des Anilinteils, sondern an einer anderen reaktiven Position des Moleküls, z. B. an einem N-Atom eines Azolyl-Restes, angreift. Außerdem ist die Bildung von tränenreizenden und stark cancerogenen Bishalogenmethylethern — die bei der technischen Durchführung des Verfahrens gemäß Reaktionsschema (2) entstehen, wenn Paraformaldehyd vorhanden ist — im Falle des erfindungsgemäßen Verfahrens nicht zu befürchten.

Verwendet man beispielsweise 2-Ethyl-6-methyl-chlor-acetanilid und N-(1-Chlormethyl)-pyrazol-hydrochlorid als Ausgangsstoffe, so kann der Reaktionsablauf des erfindungsgemäßen Verfahrens durch das folgende Formelschema wiedergegeben werden:

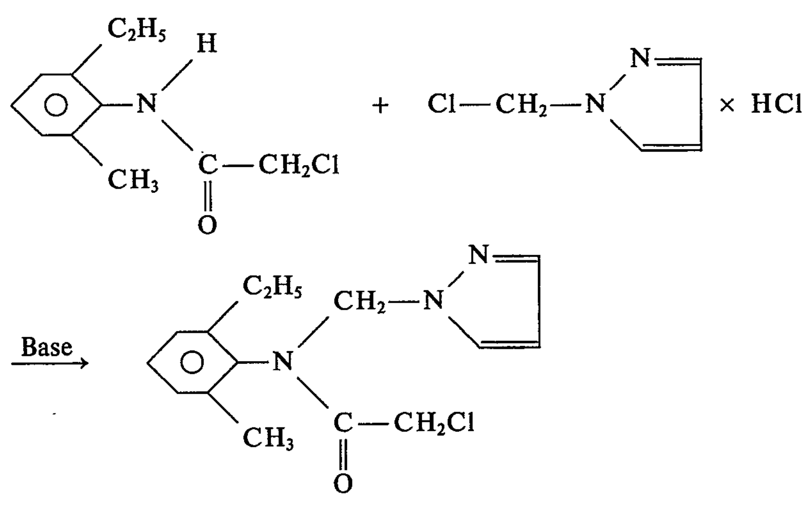


Die bei der Durchführung des erfindungsgemäßen Verfahrens als Ausgangsstoffe benötigten Halogenacetanilide sind durch die Formel (XII) allgemein definiert. In dieser Formel stehen Hal, $X^1$ und $X^2$ für diejenigen Reste, die bereits im Zusammenhang mit der Beschreibung der erfindungsgemäß herstellbaren Stoffe der Formel (XI) für diese Reste genannt wurden.

Die Halogenacetanilide der Formel (XII) sind allgemein bekannt bzw. können sie in allgemein bekannter Art und Weise erhalten werden, indem man entsprechende Aniline mit einem Halogenacethalogenid oder Halogenacetanhydrid in Gegenwart eines inerten organischen Lösungsmittels, wie beispielsweise Toluol oder Dimethylformamid, gegebenenfalls in Gegenwart eines Säurebindemittels, wie z. B. Kaliumcarbonat, bei Temperaturen zwischen 20 und 100° C umsetzt.

Als Beispiele für Verbindungen der Formel (XII) seien genannt:

Chlor(brom)acetanilid; 2-Methyl-chlor(brom)acetanilid; 2-Ethyl-chlor(brom)acetanilid; 2-Isopropyl-chlor(brom)acetanilid; 2-sek.-Butyl-chlor(brom)acetanilid; 2-tert.-Butyl-chlor(brom)acetanilid; 2,6-Dimethyl-chlor(brom)acetanilid; 2,3-Dimethyl-chlor(brom)acetanilid; 2,5-Dimethyl-chlor(brom)acetanilid; 2,6-Diethyl-chlor(brom)acetanilid; 2-Ethyl-6-methyl-chlor(brom)acetanilid.

Die außerdem für die erfindungsgemäße Umsetzung als Ausgangsstoffe zu verwendenden Verbindungen sind durch die Formel (XIII) allgemein definiert. In dieser Formel stehen R und $R^1$ für diejenigen Reste, die bereits im Zusammenhang mit der Beschreibung der erfindungsgemäß herstellbaren Stoffe der Formel (XI) für diese Reste genannt wurden.

Die Verbindungen der Formel (XIII) sind weitgehend bekannt. Noch nicht bekannt sind solche Verbindungen der Formel (XIII), in denen R für einen über ein Ring-Stickstoffatom gebundenen, gegebenenfalls substituierten heterocyclischen Rest steht und $R^1$ für Alkyl mit 1 bis 4 Kohlenstoffatomen oder Phenyl steht. Sie können erhalten werden, indem man einen Heterocyclus der Formel

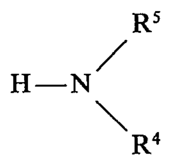

(XIV)

in welcher

$R^4$ und $R^5$ für Pyrazol-1-yl, 1,2,4-Triazol-1-yl, 1,2,3-Triazol-1-yl, 1,3,4-Triazol-1-yl, 1,2,3,4-Tetrazol-1-yl, oder Pyrrol-1-yl stehen, wobei jeder dieser Reste substituiert sein kann durch Halogen und/oder Alkyl mit 1 bis 4 Kohlenstoffatomen,

mit einem Aldehyd der Formel

$$O{=}CH{-}R^6 \qquad (XV)$$

gegebenenfalls in Gegenwart eines inerten organischen Lösungsmittels, wie beispielsweise Methylenchlorid, bei Temperaturen zwischen −70°C und +50°C umsetzt und die dabei entstehenden Verbindungen der Formel

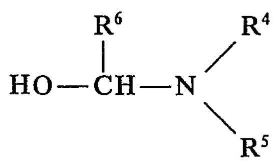

(XVI)

in welcher

$R^4$ und $R^5$ die oben angegebene Bedeutung haben, und
$R^6$ für Alkyl mit 1 bis 4 Kohlenstoffatomen oder Phenyl steht,

direkt oder gegebenenfalls nach Isolierung mit einem Chlorierungsmittel, wie beispielsweise Thionylchlorid, oder einem Sulfonylierungsmittel, wie beispielsweise Methylsulfochlorid, gegebenenfalls in Gegenwart eines inerten organischen Lösungsmittels, wie z. B. Methylenchlorid, bei Temperaturen zwischen −70° C und +50° C umsetzt.

Die Heterocyclen der Formel (XIV) sind bekannt.

Als Beispiele für Verbindungen der Formel (XIII) seien im einzelnen genannt:

1-(1′-Brom(chlor)ethyl)-pyrazol
1-(1′-Brom(chlor)ethyl)-4-chlor-pyrazol
1-(1′-Brom(chlor)ethyl)-2-methyl-pyrazol
1-(1′-Brom(chlor)ethyl)-5-methyl-pyrazol
1-(1′-Brom(chlor)ethyl)-3,5-dimethyl-pyrazol
1-(1′-Brom(chlor)ethyl)-4-chlor-3,5-dimethyl-pyrazol
1-(1′-Brom(chlor)propyl)-pyrazol
1-(1′-Brom(chlor-propyl)-4-chlor-pyrazol
1-(1′-Brom(chlor)propyl)-3-methyl-pyrazol
1-(1′-Brom(chlor)propyl)-5-methyl-pyrazol

1-(1'-Brom(chlor)propyl)-3,5-dimethyl-pyrazol
1-(1'-Brom(chlor)propyl)-4-chlor-3,5-dimethyl-pyrazol
1-(1'-Brom(chlor)butyl)-pyrazol
1-(1'-Brom(chlor)butyl)-4-chlorpyrazol
1-(1'-Brom(chlor)butyl)-1-methyl-pyrazol
1-(1'-Brom(chlor)butyl)-5-methyl-pyrazol
1-(1'-Brom(chlor)butyl)-3,5-dimethyl-pyrazol
1-(1'-Brom(chlor)butyl)-4-chlor-3,5-dimethyl-pyrazol
1-(1'-Brom(chlor)pentyl)-pyrazol
1-(1'-Brom(chlor)pentyl)-4-chlor-pyrazol
1-(1'-Brom(chlor)pentyl)-3-methyl-pyrazol
1-(1'-Brom(chlor)pentyl)-5-methylpyrazol
1-(1'-Brom(chlor)pentyl)-3,5-dimethyl-pyrazol
1-(1'-Brom(chlor)pentyl)-4-chlor-3,5-dimethyl-pyrazol

Als Lösungsmittel kommen für die erfindungsgemäße Umsetzung alle inerten organischen Lösungsmittel infrage. Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die Reaktionstemperaturen können bei der Durchführung des erfindungsgemäßen Verfahrens in einem größeren Bereich variiert werden. Vorzugsweise arbeitet man zwischen 0° C und 80° C.

Bei der Durchführung des erfindungsgemäßen Verfahrens setzt man vorzugsweise auf 1 bis 1,5 Mol Halogenacetanilid der Formel (XII) bis 1,5 Mol der Verbindung der Formel (XIII) ein. Die Isolierung der Verbindungen der Formel (XI) erfolgt in üblicher Weise.

In einer bevorzugten Ausführungsform wird die erfindungsgemäße Umsetzung in einem Zweiphasensystem, wie beispielsweise wäßrige Natron- oder Kalilauge/Toluol oder Methylenchlorid, unter Zusatz von 0,01 – 1 Mol eines Phasen-Transfer-Katalysators, wie beispielsweise einer Ammonium- oder Phosphoniumverbindung, beispielsweise seien Benzyldodecyl-dimethyl-ammoniumchlorid (Zephirol) und Triethylbenzyl-ammoniumchlorid genannt, durchgeführt.

Bei der Durchführung dieser Variante des erfindungsgemäßen Verfahrens setzt man vorzugsweise auf 1 bis 1,5 Mol Halogenacetanilid der Formel (XII) 1 bis 1,5 Mol Verbindung der Formel (XIII) sowie 1 bis 10 Mol Säurebinder ein. Die Isolierung der Verbindungen der Formel (XI) erfolgt in üblicher Weise.

Die erfindungsgemäß herstellbaren Wirkstoffe der Formel (XI) zeichnen sich bekanntermaßen durch sehr gute herbizide und auch fungizide Wirksamkeit aus (vgl. die im Rahmen oder Abhandlung des bekannten Standes der Technik angegebenen US-Patentschriften und deutschen Offenlegungsschriften).

Das erfindungsgemäße Verfahren sei anhand der folgenden Herstellungsbeispiele erläutert.

## Herstellungsbeispiele

### Beispiel 1

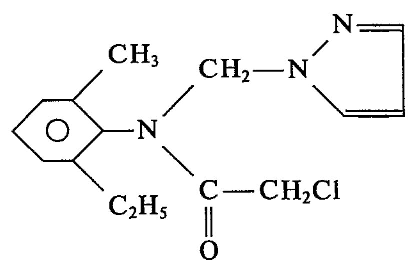

213 g (1 Mol) 2-Ethyl-6-methyl-chloracetanilid und 4 ml Benzyl-dodecyl-dimethylammoniumchlorid werden in einem 2-Phasen-Gemisch aus 400 g Natriumhydroxid in 400 ml Wasser und 800 ml Methylenchlorid gelöst und bei 20°C bis 25°C innerhalb 1 Stunde mit 170 g (1,1 Mol) N-Chlormethylpyrazol-hydrochlorid versetzt. Es wird 3 Stunden bei 25°C nachgerührt, anschließend mit 500 ml Wasser versetzt, die organische Phase abgetrennt, mehrfach mit Wasser gewaschen, durch Zugabe von Zitronensäure auf pH 3 gestellt und nochmals mit Wasser gewaschen, über Natriumsulfat getrocknet und im Vakuum eingeengt. Man erhält 247 g (85% der Theorie) N-Pyrazol-1-yl-methyl-2-ethyl-6-methyl-chloracetanilid vom Schmelzpunkt 57° C.

Beispiel 2

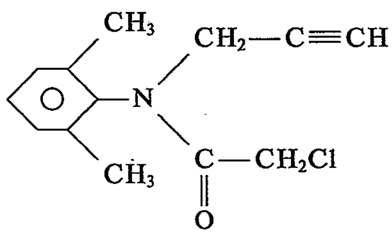

19,8 g (0,1 Mol) 2,6-Dimethyl-chloracetanilid und 0,5 g Triethylbenzylammoniumchlorid werden in einem 2-Phasen-Gemisch aus 50 ml 50%iger Natronlauge und 150 ml Toluol gelöst und bei 20 bis 30° C unter heftigem Rühren tropfenweise mit 13,1 g (0,11 Mol) Propargylbromid versetzt. Es wird noch 2 Stunden nachgerührt, anschließend die organische Phase mehrmals mit Wasser gewaschen, über Natriumsulfat getrocknet und im Vakuum eingeengt. Das kristalline Rohprodukt wird aus Cyclohexan umkristallisiert. Man erhält 16,8 g (71,5% der Theorie) N-Propargyl-2,6-dimethyl-chloracetanilid vom Schmelzpunkt 82–84° C.

Beispiel 3

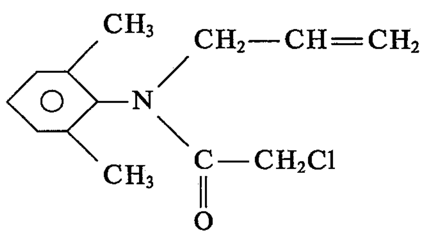

118,5 g (0,6 Mol) 2,6-Dimethyl-chloracetanilid und 1 g Triethylbenzylammoniumchlorid werden in einem 2-Phasen-Gemisch aus 300 ml 50%iger Natronlauge und 750 ml Toluol gelöst und bei 20 bis 30° C unter heftigem Rühren mit 79,8 g (0,66 Mol) Allylbromid tropfenweise versetzt. Es wird noch 3 Stunden nachgerührt, anschließend die organische Phase mehrmals mit Wasser gewaschen, über Natriumsulfat getrocknet und eingeengt. Man erhält 115 g (81% der Theorie) N-Allyl-2,6-dimethyl-chloracetanilid vom Schmelzpunkt 62–67° C.

Beispiel 4

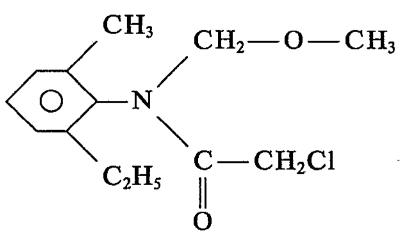

21,3 g (0,1 Mol) 2-Ethyl-6-methyl-chloracetanilid und 1 ml Benzyl-dodecyl-dimethylammoniumchlorid werden in einem 2-Phasen-Gemisch aus 50 ml 50%iger Kalilauge und 50 ml Methylenchlorid gelöst und bei 0° C unter Rühren mit 8 g (0,1 Mol) Monochlordimethylether versetzt. Man läßt die Temperatur auf 25° C ansteigen und rührt 12 Stunden bei dieser Temperatur. Anschließend wird die organische Phase abgetrennt und die Wasserphase dreimal mit je 50 ml Methylenchlorid ausgeschüttelt. Die vereinigten organischen Phasen werden nochmals mit Wasser gewaschen, über Natriumsulfat getrocknet und durch Abdestillieren des Lösungsmittels im Vakuum eingeengt. Der Rückstand wird mit 100 ml Cyclohexan und dann mit 200 ml Petrolether versetzt, eventuelle Neben- und noch vorhandene Ausgangsprodukte werden jeweils abfiltriert und das Filtrat eingeengt. Der Rückstand wird destilliert. Man erhält so 18,4 g (72,2% der Theorie) N-Methoxymethyl-2-ethyl-6-methyl-chloracetanilid vom Siedepunkt 121° C/0,1 mm.

Beispiel 5

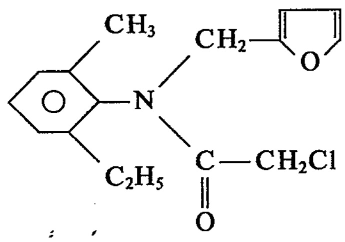

10 g (0,047 Mol) 2-Ethyl-6-methyl-chloracetanilid und 1 ml Benzyl-dodecyl-dimethylammoniumchlorid werden in einem 2-Phasen-Gemisch aus 50 ml 50%iger Kalilauge und 50 ml Methylenchlorid gelöst und bei 0°C unter Rühren mit 6 g (0,052 Mol) 2-Chlormethyl-furan versetzt. Man läßt die Temperatur auf 25°C ansteigen und rührt 12 Stunden bei dieser Temperatur. Anschließend wird die organische Phase abgetrennt und die Wasserphase dreimal mit je 50 ml Methylenchlorid ausgeschüttelt. Die vereinigten Phasen werden nochmals zweimal mit je 30 ml Wasser gewaschen, über Natriumsulfat getrocknet und durch Abdestillation des Lösungsmittels im Vakuum eingeengt. Der Rückstand wird mit 100 ml Cyclohexan und dann mit 200 ml Petrolether versetzt, eventuelle Neben- und noch vorhandene Ausgangsprodukte werden jeweils abfiltriert und das Filtrat eingeengt. Man erhält 10,6 g (77% der Theorie) N-(Fur-2-methyl)-2-ethyl-6-methyl-chloracetanilid als zähflüssiges Öl.

Beispiel 6

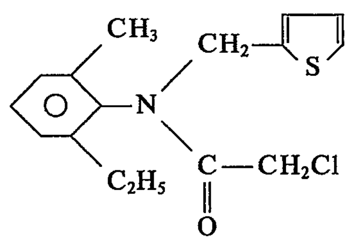

10 g (0,047 Mol) 2-Ethyl-6-methyl-chloracetanilid und 1 ml Benzyl-dodecyl-dimethylammoniumchlorid werden in einem 2-Phasen-Gemisch aus 50 ml 50%iger Kalilauge und 50 ml Methylenchlorid gelöst und bei 0°C unter Rühren mit 6,5 g (0,05 Mol) 2-Chlormethyl-thiophen versetzt. Man läßt die Temperatur auf 25°C ansteigen und rührt 12 Stunden bei dieser Temperatur. Anschließend wird die organische Phase abgetrennt und die Wasserphase dreimal mit je 50 ml Methylenchlorid ausgeschüttelt. Die vereinigten organischen Phasen werden nochmals mit Wasser gewaschen, über Natriumsulfat getrocknet und durch Abdestillieren des Lösungsmittels im Vakuum eingeengt. Der Rückstand wird mit je 100 ml Cyclohexan und dann mit 200 ml Petrolether versetzt, eventuelle Neben- und noch vorhandene Ausgangsprodukte werden jeweils abfiltriert und das Filtrat eingeengt. Der Rückstand wird mehrmals mit Ligroin behandelt. Die vereinigten Ligroinphasen werden über Natriumsulfat getrocknet und im Vakuum eingeengt. Man erhält 11,9 g (82,5% der Theorie) N-(Thiophen-2-yl-methyl)-2-ethyl-6-methyl-chloracetanilid als zähflüssiges Öl.

Beispiel 7

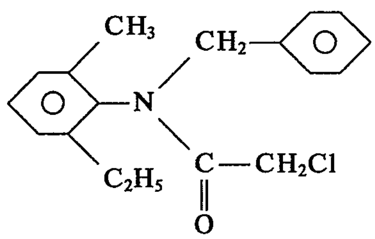

42,2 g (0,2 Mol) 2-Ethyl-6-methyl-chloracetanilid und 1 ml Benzyl-dodecyl-dimethylammoniumchlorid werden in einem 2-Phasen-Gemisch aus 100 ml 50%iger Kalilauge und 100 ml Methylenchlorid gelöst und bei 0°C unter Rühren mit 34,5 g Benzylbromid (0,2 Mol) versetzt. Man läßt die Temperatur auf 25°C ansteigen und rührt 12 Stunden bei dieser Temperatur. Anschließend wird die organische Phase abgetrennt und die Wasserphase 3× mit je 50 ml Methylenchlorid ausgeschüttelt. Die vereinigten organischen Phasen werden nochmals mit 2× 30 ml Wasser gewaschen, über

Natriumsulfat getrocknet und durch Abdestillieren des Lösungsmittels im Vakuum eingeengt. Den Rückstand versetzt man mit 300 ml Petrolether. Eventuelle Nebenprodukte und noch vorhandenes Ausgangsmaterial werden abfiltriert und das Filtrat eingeengt. Man erhält 51,8 g (86% der Theorie) N-(Benzyl-2-ethyl-6-methyl-chloracetanilid als zähflüssiges Öl.

In analoger Weise werden die in der folgenden Tabelle formelmäßig aufgeführten Verbindungen hergestellt.

Tabelle 1

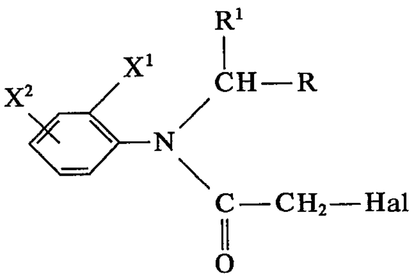

(XI)

| Beispiel Nr. | $X^1$ | $X^2$ | R | $R^1$ | Hal | Fp (°C) |
|---|---|---|---|---|---|---|
| 8 | $CH_3$ | 6-$C_2H_5$ | 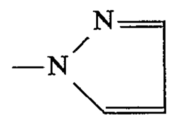 | $CH_3$ | Cl | 87 |
| 9 | $CH_3$ | 6-$CH_3$ | 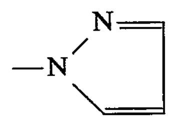 | $CH_3$ | Cl | 90 |
| 10 | $C_2H_5$ | 6-$C_2H_5$ | 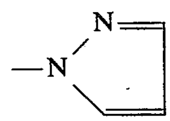 | $CH_3$ | Cl | 80 |
| 11 | $C_2H_5$ | 6-$C_2H_5$ | 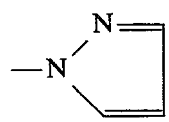 | H | Cl | 67 |
| 12 | $C_2H_5$ | 6-$C_2H_5$ | 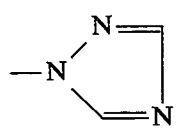 | H | Cl | 112 |
| 13 | i-$C_3H_7$ | H | 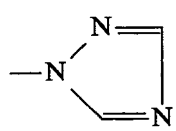 | H | Cl | 118 |
| 14 | i-$C_3H_7$ | H | 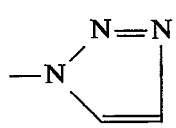 | H | Cl | Öl |
| 15 | $CH_3$ | 6-$C_2H_5$ | 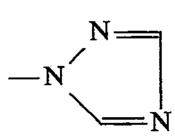 | H | Cl | Öl |

Tabelle 1 (Fortsetzung)

| Beispiel Nr. | $X^1$ | $X^2$ | R | $R^1$ | Hal | Fp (°C) |
|---|---|---|---|---|---|---|
| 16 | $C(CH_3)_3$ | H | 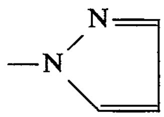 | H | Cl | Öl |
| 17 | $C(CH_3)_3$ | H | 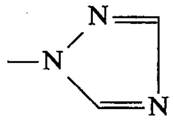 | H | Cl | 118 |
| 18 | $C_2H_5$ | 6-$C_2H_5$ | 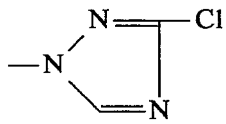 | H | Cl | 110 |
| 19 | $CH_3$ | 6-$C_2H_5$ | 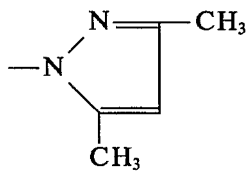 | H | Cl | 90 |
| 20 | $C_2H_5$ | 6-$C_2H_5$ | 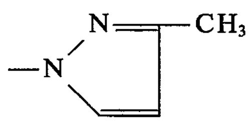 | H | Cl | 89 |
| 21 | $CH_3$ | 6-$C_2H_5$ | 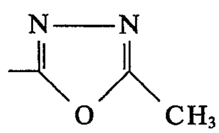 | H | Cl | 67–70 |
| 22 | $C_2H_5$ | 6-$C_2H_5$ | 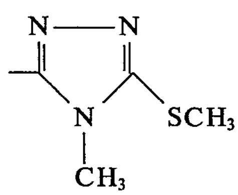 | H | Cl | 121–23 |
| 23 | $C_2H_5$ | 6-$C_2H_5$ | 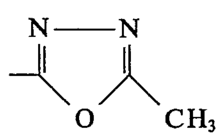 | H | Cl | 79–82 |
| 24 | $CH_3$ | 6-$CH_3$ | 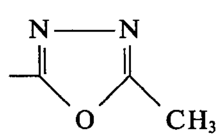 | H | Cl | 91–93 |
| 25 | $C(CH_3)_3$ | H | 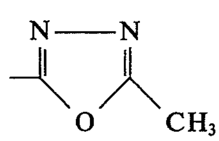 | H | Cl | 102–04 |

Tabelle 1 (Fortsetzung)

| Beispiel Nr. | $X^1$ | $X^2$ | R | $R^1$ | Hal | Fp (°C) |
|---|---|---|---|---|---|---|
| 26 | $C_2H_5$ | 6-$C_2H_5$ | 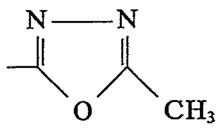 | H | Br | 80 |
| 27 | $CH_3$ | H | 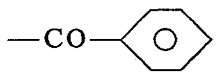 | H | Cl | 138 |
| 28 | $C_2H_5$ | 6-$C_2H_5$ | 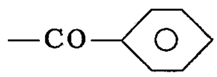 | H | Cl | 116 |
| 29 | $CH_3$ | 6-$CH_3$ | 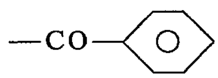 | H | Cl | 100 |
| 30 | $CH_3$ | 6-$CH_3$ | $-CO-CH_3$ | $CH_3$ | Cl | 104 |
| 31 | $CH_3$ | 6-$CH_3$ | 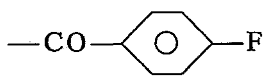 | H | Cl | 104 |
| 32 | $C_2H_5$ | 6-$C_2H_5$ | $-OCH_3$ | H | Cl | 40–41 |

Herstellung von Verbindungen der Formel (XII)

Beispiel XII-1

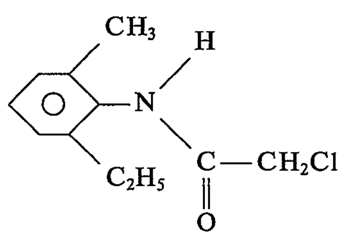

135,2 g (1 Mol) 2-Ethyl-6-methyl-anilin in 1000 ml Toluol werden mit 152 g (1,1 Mol) Kaliumcarbonat versetzt. Dazu werden unter Rühren 113 g (1 Mol) Chloressigsäurechlorid getropft. Nach Abklingen der exothermen Reaktion läßt man 2 Stunden unter Rückfluß nachrühren. Anschließend wird das Reaktionsgemisch filtriert und das Filtrat im Vakuum auf 500 ml eingeengt. Die dabei entstehenden Kristalle werden abgesaugt und mit Petrolether gewaschen. Man erhält 189,6 g (98% der Theorie) 2-Ethyl-6-methyl-chloracetanilid in Form weißer Kristelle vom Schmelzpunkt 120° C.

In analoger Weise werden die in der nachstehenden Tabelle 2 formelmäßig aufgeführten Verbindungen der Formel (XII) erhalten.

Tabelle 2

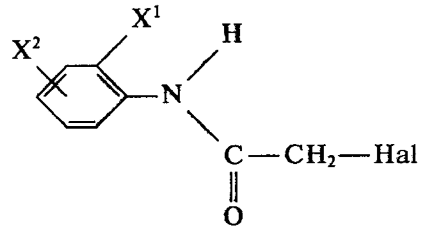

(XII)

| Beispiel Nr. | $X^1$ | $X^2$ | Hal | Schmelzpunkt (°C) |
|---|---|---|---|---|
| XII-2 | $CH_3$ | 6-$CH_3$ | Cl | 148 |
| XII-3 | $C_2H_5$ | 6-$C_2H_5$ | Cl | 133 |
| XII-4 | i-$C_3H_7$ | H | Cl | 79 |
| XII-5 | tert.-$C_4H_9$ | H | Cl | 96 |
| XII-6 | $C_2H_5$ | H | Cl | 103 |
| XII-7 | $CH_3$ | H | Cl | 109 |
| XII-8 | $CH_3$ | 3-$CH_3$ | Cl | 135 |
| XII-9 | $CH_3$ | 5-$CH_3$ | Cl | 154 |
| XII-11 | sek.-$C_4H_9$ | H | Cl | Öl |
| XII-12 | H | H | Cl | 132 |

### Herstellung von Verbindungen der Formel (XIII)

#### Beispiel XIII-1

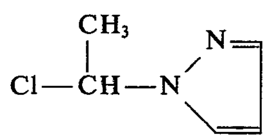

× HCl

Zu 340 g (5 Mol) Pyrazol in 1200 ml Methylenchlorid werden bei 0 bis 5°C innerhalb einer Stunde 250 g (5,7 Mol) Acetaldehyd getropft. Man läßt ca. 1 Stunde bei 0°C nachrühren. Das dabei entstehende N-(1-Hydroxyethyl)-pyrazol wird nicht isoliert, sondern die Reaktionslösung wird direkt bei 0 bis 5°C innerhalb einer Stunde zu 1250 g (10,5 Mol) Thionylchlorid getropft. Man läßt 1 Stunde bei 20°C nachrühren und engt dann bei 40°C im Vakuum ein. Nach Zugabe von 300 ml Methylenchlorid wird erneut eingeengt. Der Rückstand wird im Vakuum destilliert. Man erhält 620 g (75% der Theorie) N-(1-Chlorethyl)-pyrazol-hydrochlorid vom Siedepunkt 55°C/18 mm.

Beispiel XIII-2

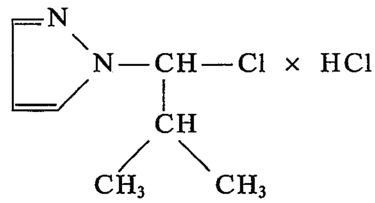

An 204 g (3 Mol) Pyrazol, gelöst in 400 ml Methylenchlorid, werden bei 0–5°C 220 g (3,2 Mol) Isobutylaldehyd zugetropft. Man läßt nach beendeter Zugabe noch zwei Stunden bei 0°C nachrühren und tropft dann die Lösung bei 0–5°C zu 750 g (6,3 Mol) Thionylchlorid. Man läßt die Reaktionsmischung anschließend auf Raumtemperatur erwärmen und rührt noch 12 Stunden nach. Danach wird das Lösungsmittel im Vakuum abgezogen, der Rückstand in Tetrachlorkohlenstoff aufgenommen und kristallisiert. Die Kristalle werden abfiltriert, die Mutterlauge eingeengt und der Rückstand kristallisiert. Der so erhaltene Feststoff wird mit der ersten Fraktion vereinigt und mit 200 ml Tetrachlorkohlenstoff ausgerührt. Danach filtriert man ab, wäscht mit 100 ml Tetrachlorkohlenstoff nach und trocknet den Feststoff bei 40°C im Vakuum. Man erhält 530 g 1-(1'-Chlor-isobutyl)-pyrazol-hydrochlorid (91% der Theorie) vom Schmelzpunkt 110–114°C.

Tabelle 3

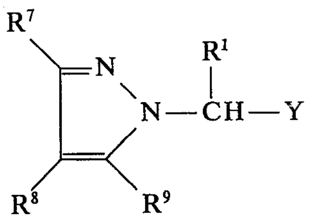

(XIII)

| Beispiel Nr. | $R^1$ | $R^7$ | $R^8$ | $R^9$ | Y | Schmelzpunkt (°C) Siedepunkt (°C) |
|---|---|---|---|---|---|---|
| XIII 3 | $-CH_3$ | $CH_3$ | Cl | $CH_3$ | Cl | Öl (× HCl) |
| XIII 4 | $-CH_3$ | H | Cl | H | Cl | Sdp.: 60–62/20 mb (× HCl) |
| XIII 5 | $-C_2H_5$ | H | H | H | Cl | Öl (× HCl) |
| XIII 6 | $-n\text{-}C_3H_7$ | H | H | H | Cl | Öl (× HCl) |
| XIII 7 | $-CH_3$ | H | H | H | Br | Sdp.: 60–65/20 mb |
| XIII 8 | $-CH_3$ | H | H | H | Cl | Sdp.: 50–55/18 mb |

**Patentansprüche**

1. Verfahren zur Herstellung von N-substituierten α-Halogenacetaniliden der Formel

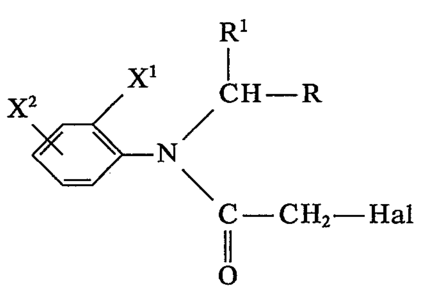

(XI)

in welcher

R für Pyrazol-1-yl, 1,2,4-Triazol-1-yl, 1,2,3-Triazol-1-yl, 1,3,4-Triazol-1-yl, 1,2,3,4-Tetrazol-1-yl oder Pyrrol-1-yl steht, wobei jeder dieser Reste substituiert sein kann durch Halogen und/oder Alkyl mit 1 bis 4 Kohlenstoffatomen, und

R ferner für die Gruppierung der Formel

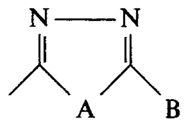

steht, in welcher

A für Sauerstoff, Schwefel oder $>NR^2$ steht,
worin
$R^2$ für Wasserstoff, Alkyl mit 1 bis 4 Kohlenstoffatomen oder Phenyl steht,
und

B für Wasserstoff, Alkyl mit 1 bis 4 Kohlenstoffatomen, Halogenalkyl mit bis zu 3 Kohlenstoffatomen und bis zu 5 gleichen oder verschiedenen Halogenatomen, Halogen, Phenyl, Benzyl oder für die Gruppierungen $-OR^3$, $-SR^3$ oder $-NR^2R^3$ steht,
worin
$R^2$ die oben angegebenen Bedeutungen hat und
$R^3$ für Wasserstoff oder Alkyl mit 1 bis 4 Kohlenstoffatomen steht,
und

R außerdem für Furyl, Thiophenyl, Alkenyl mit 2 bis 4 Kohlenstoffatomen, Alkinyl mit 2 bis 4 Kohlenstoffatomen, Alkoxy mit 1 bis 4 Kohlenstoffatomen, Alkylcarbonyl mit 1 bis 4 Kohlenstoffatomen im Alkylteil oder für Phenyl, Phenoxy oder Phenylcarbonyl steht, wobei jeder der drei letztgenannten Reste substituiert sein kann durch Halogen und/oder Alkyl mit 1 bis 4 Kohlenstoffatomen,

$R^1$ für Wasserstoff, Alkyl mit 1 bis 4 Kohlenstoffatomen oder Phenyl steht,

$X^1$ für Alkyl mit 1 bis 4 Kohlenstoffatomen steht,

$X^2$ für Wasserstoff oder Alkyl mit 1 bis 4 Kohlenstoffatomen steht und

Hal für Chlor oder Brom steht,

dadurch gekennzeichnet, daß man Halogenacetanilide der Formel

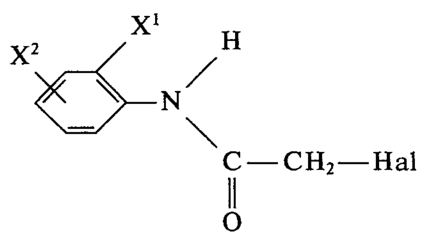

(XII)

in welcher

$X^1$, $X^2$
und Hal die oben angegebene Bedeutung haben,

mit Verbindungen der Formel

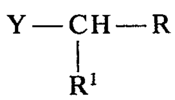

(XIII)

in welcher

R und $R^1$ die oben angegebene Bedeutung haben und

Y für Chlor, Brom, Jod, Alkylsulfonyl mit 1 bis 4 Kohlenstoffatomen oder für gegebenenfalls durch Halogen oder Alkyl mit 1 bis 4 Kohlenstoffatomen substituiertes Phenylsulfonyl steht,

in Gegenwart eines Alkalihydroxids oder eines Alkalicarbonats als Säurebinder und gegebenenfalls in Gegenwart eines organischen Lösungsmittels bei Temperaturen zwischen $-20°C$ und $+100°C$

umsetzt, wobei die Verbindungen der Formel (XIII) gegebenenfalls in Form von Säureadditions-Salzen umgesetzt werden können.

2. Verfahren gemäß Anspruch 1, dadurch gekennzeichnet, daß man auf 1 bis 1,5 Mol Halogenacetanilid der Formel (XII) 1 bis 1,5 Mol einer Verbindung der Formel (XIII) einsetzt.

3. Verfahren gemäß Anspruch 1, dadurch gekennzeichnet, daß man in einem Zweiphasensystem in Gegenwart eines Phasen-Transfer-Katalysators arbeitet.

4. Verfahren gemäß Ansprüchen 1 und 3, dadurch gekennzeichnet, daß man Ammonium- oder Phosphonium-Verbindungen als Phasen-Transfer-Katalysatoren einsetzt.

5. Verfahren gemäß Ansprüchen 1, 3 und 4, dadurch gekennzeichnet, daß man Triethyl-benzyl-ammonium-chlorid oder Benzyl-dodecyl-dimethyl-ammoniumchlorid als Phasen-Transfer-Katalysatoren einsetzt.

## Claims

1. Process for the preparation of N-substituted α-halogenoacetanilides of the formula

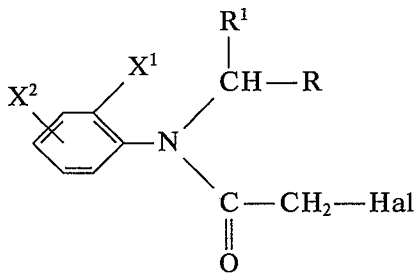

(XI)

in which

R represents pyrazol-1-yl, 1,2,4-triazol-1-yl, 1,2,3-triazol-1-yl, 1,3,4-triazol-1-yl, 1,2,3,4-tetrazol-1-yl or pyrrol-1-yl, it being possible for each of these radicals to be substituted by halogen and/or alkyl with 1 to 4 carbon atoms and

R also represents the grouping

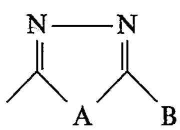

in which

A represents oxygen, sulphur or $>NR^2$, wherein

$R^2$ represents hydrogen, alkyl with 1 to 4 carbon atoms or phenyl, and

B represents hydrogen, alkyl with 1 to 4 carbon atoms, halogenoalkyl with up to 3 carbon atoms and up to 5 identical or different halogen atoms, halogen, phenyl, benzyl or the groupings $-OR^3$, $-SR^3$ or $-NR^2R^3$, wherein

$R^2$ has the meanings given above and

$R^3$ represents hydrogen or alkyl with 1 to 4 carbon atoms, and

R furthermore represents furyl, thiophenyl, alkenyl with 2 to 4 carbon atoms, alkynyl with 2 to 4 carbon atoms, alkoxy with 1 to 4 carbon atoms, alkylcarbonyl with 1 to 4 carbon atoms in the alkyl part or phenyl, phenoxy or phenylcarbonyl, it being possible for each of the three last-mentioned radicals to be substituted by halogen and/or alkyl with 1 to 4 carbon atoms,

$R^1$ represents hydrogen, alkyl with 1 to 4 carbon atoms or phenyl,

$X^1$ represents alkyl with 1 to 4 carbon atoms,

$X^2$ represents hydrogen or alkyl with 1 to 4 carbon atoms and

Hal represents chlorine or bromine,

characterised in that halogenoacetanilides of the formula

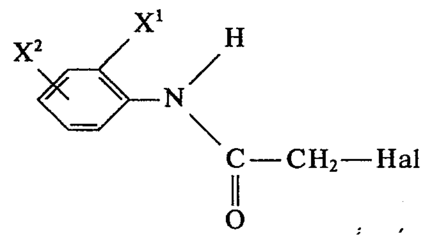 (XII)

in which

$X^1$, $X^2$ and Hal have the meaning indicated above,

are reacted with compounds of the formula

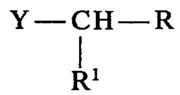 (XIII)

in which

R and $R^1$ have the meaning indicated above and
Y represents chlorine, bromine, iodine, alkylsulphonyl with 1 to 4 carbon atoms or phenylsulphonyl optionally substituted by halogen or alkyl with 1 to 4 carbon atoms,

in the presence of an alkali metal hydroxide or an alkali metal carbonate as acid binder and optionally in the presence of an organic solvent, at temperatures between $-20°C$ and $+100°C$, it being possible optionally to react the compounds of the formula (XIII) in the form of acid addition salts.

2. Process according to Claim 1, characterised in that 1 to 1.5 mols of a compound of the formula (XIII) are used per 1 to 1.5 mols of the halogenacetanilide of the formula (XII).

3. Process according to Claim 1, characterised in that the reaction is carried out in a two-phase system in the presence of a phase transfer catalyst.

4. Process according to Claims 1 and 3, characterised in that ammonium or phosphonium compounds are used as phase transfer catalysts.

5. Process according to Claims 1, 3 and 4, characterised in that triethyl-benzyl-ammonium chloride or benzyl-dodecyl-dimethyl-ammonium chloride are used as phase transfer catalysts.

**Revendications**

1. Procédé de préparation d'$\alpha$-halogénacétanilides N-substitués de formule:

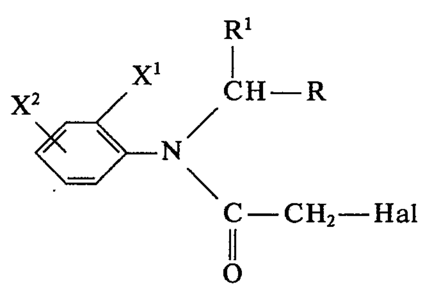 (XI)

dans laquelle

R représente un groupe pyrazol-1-yle, un groupe 1,2,4-triazol-1-yle, un groupe 1,2,3-triazol-1-yle, un groupe 1,3,4-triazol-1-yle, un groupe 1,2,3,4-tétrazol-1-yle ou un groupe pyrrol-1-yle, chacun de ces radicaux pouvant être substitué par un atome d'halogène et/ou par un groupe alkyle contenant 1 à 4 atomes de carbone, et

R représente également le groupement de formule

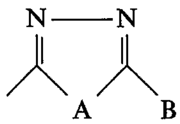

dans laquelle

A représente un atome d'oxygène, un atome de soufre ou le groupe >$NR^2$,

où

$R^2$ représente un atome d'hydrogène, un groupe alkyle contenant 1 à 4 atomes de carbone, ou un groupe phényle,

et

B représente un atome d'hydrogène, un groupe alkyle contenant 1 à 4 atomes de carbone, un groupe halogénalkyle contenant jusqu'à 3 atomes de carbone et jusqu'à 5 atomes d'halogènes identiques ou différents, un atome d'halogène, un groupe phényle, un groupe benzyle ou les groupements $-OR^3$, $-SR^3$ ou $-NR^2R^3$,

ou

$R^2$ a les significations indiquées ci-dessus, et

$R^3$ représente un atome d'hydrogène ou un groupe alkyle contenant 1 à 4 atomes de carbone,

et

R représente également un groupe furyle, un groupe thiophényle, un groupe alcényle contenant 2 à 4 atomes de carbone, un groupe alcinyle contenant 2 à 4 atomes de carbone, un groupe alcoxy contenant 1 à 4 atomes de carbone, un groupe alkylcarbonyle contenant 1 à 4 atomes de carbone dans la fraction alkyle, ou un groupe phényle, un groupe phénoxy ou un groupe phénylcarbonyle, chacun des trois derniers groupes pouvant être substitué par un atome d'halogène et/ou par un groupe alkyle contenant 1 à 4 atomes de carbone,

$R^1$ représente un atome d'hydrogène, un groupe alkyle contenant 1 à 4 atomes de carbone, ou un groupe phényle,

$X^1$ représente un groupe alkyle contenant 1 à 4 atomes de carbone,

$X^2$ représente un atome d'hydrogène ou un groupe alkyle contenant 1 à 4 atomes de carbone, et

Hal représente un atome de chlore ou un atome de brome,

caractérisé en ce qu'on fait réagir des halogénacétanilides de formule:

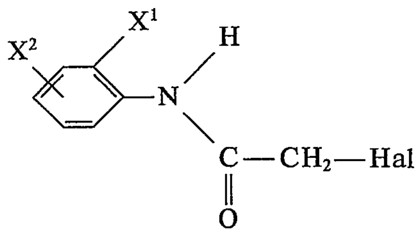

(XII)

dans laquelle

$X^1$, $X^2$ et Hal. ont les significations indiquées ci-dessus,

avec des composés de formule:

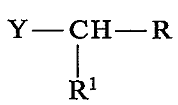

(XIII)

dans laquelle

R et $R^1$ ont les significations indiquées ci-dessus, et

Y représente un atome de chlore, un atome de brome, un atome d'iode, un groupe alkyl-sulfonyle contenant 1 à 4 atomes de carbone, ou un groupe phényl-sulfonyle éventuellement substitué par un atome d'halogène ou par un groupe alkyle contenant 1 à 4 atomes de carbone,

en présence d'un hydroxyde alcalin ou d'un carbonate alcalin comme fixateur d'acide et

èventuellement en présence d'un solvant organique, à des températures comprises entre −20°C et +100°C, les composés de formule (XIII) pouvant èventuellement être mis à réagir sous forme de sels d'addition d'acide.

2. Procédé suivant la revendication 1, caractérisé en ce que, pour 1 à 1,5 mole d'halogénacétanilide de formule (XII), on utilise 1 à 1,5 mole d'un composé de formule (XIII).

3. Procédé suivant la revendication 1, caractérisé en ce qu'on travaille dans un système à deux phases en présence d'un catalyseur de transfert de phases.

4. Procédé suivant les revendications 1 et 3, caractérisé en ce que, comme catalyseurs de transfert de phases, on utilise des composés d'ammonium ou des composés de phosphonium.

5. Procédé suivant les revendications 1, 3 et 4, caractérisé en ce que, comme catalyseurs de transfert de phases, on utilise le chlorure de triéthyl-benzyl-ammonium ou le chlorure de benzyl-dodécyl-diméthyl-ammonium.